# EUROPEAN PATENT APPLICATION

(11) **EP 4 848 305 A1**
(43) Date of publication of application: **02.12.2026**
(21) Application number: 25179383.2
(22) Date of filing: 28.05.2025
(51) Int. Cl.: G16H 40/67, G16H 50/20, G16H 50/30, G16H 50/70

(54) **SUBJECT MONITORING**

(71) Applicant: Koninklijke Philips N.V., 5656 AE Eindhoven (NL)
(72) Inventor: BEZWORK, Yamiral, Eindhoven (NL); PAPINI, Gabriele, Eindhoven (NL); COX, Lieke Gertruda Elisabeth, Eindhoven (NL)
(74) Representative: Philips Intellectual Property & Standards

(57) **Abstract**

A mechanism for predicting a plurality of mobility metrics for a subject. Sensor data, produced by sensors supported by an upper body of the subject, is processed using a multi-task learning model to predict the plurality of mobility metrics.

## Description

### FIELD OF THE INVENTION

The present invention relates to the field of subject monitoring.

### BACKGROUND OF THE INVENTION

There is a well-established link between subject activity levels and adverse outcomes (e.g., low mobility levels, functionality and/or risk of mortality), particularly in clinical settings such as hospitals. In particular, (extremely) low activity levels of a subject have been linked to adverse outcomes.

In addition, it has been shown that, at least in clinical settings, monitoring subject activity (e.g., in combination with vital signs) helps to provide a more comprehensive understanding of patient recovery and enable timely clinical interventions.

There is therefore a need to identify and/or quantify a subject's behavior and activities. Typically, this is performed via direct observation by clinical staff or self-reported questionnaires. However, direct observation is time-consuming and subjective, whilst self-reported questionnaires rely on patient recall, making them prone to reporting biases and subjective misjudgments.

To address these shortcomings, wearable activity monitors have emerged to enable objective and continuous tracking of activity metrics and other mobility metrics. Wearable activity monitors derive activity metrics from signals acquired from sensors like accelerometers, gyroscopes, and barometers. The signal(s) may then be processed to predict a measure of an activity level or another subject property.

There is an ongoing demand for accurate monitoring of one or more mobility metrics (e.g., activity metrics) within a variety of environments, such as clinical settings and home settings.

### SUMMARY OF THE INVENTION

The invention is defined by the claims.

According to examples in accordance with an aspect of the invention, there is provided a computer-implemented method for generating a plurality of mobility metrics for a subject. The method includes obtaining sensor data produced by one or more sensors supported by an upper body of the subject, and processing the sensor data using a single multi-task machine-learning model, trained using a multi-task learning technique, to produce the plurality of mobility metrics for the subject, wherein each mobility metric corresponds to a different task of the multi-task machine-learning model.

This proposed approach provides a mechanism for generating multiple different mobility metrics from a single set of sensor data using a unified machine-learning model. By exploiting multi-task learning, the approach reduces computational overhead and allows for more comprehensive mobility analysis from upper body sensor data. The proposed approach also recognizes that there is an overlap or influence between different mobility metrics. Thus, training a machine-learning model to accurately predict one form of mobility metric is likely to inherently improve the model's ability to predict other related mobility metrics. This synergistic effect thereby enhances the overall accuracy of mobility metric predictions.

In some examples, the one or more sensors are supported by a neck and/or torso of the subject.

It will be appreciated that a mobility metric may be understood as a measurement or indication of a physical property of the subject, thereby providing quantitative or qualitative information about a respective aspect of the subject's movement, posture, or overall physical state. The present disclosure recognizes that there is a factual relationship between sensor data produced by sensors supported or worn by an upper body of the subject and each mobility metric.

The sensor data may, for instance, comprise physical activity information that captures and represents the subject's physical movements and behaviors. The sensor data may, for instance, comprise motion data (an example of physical activity information) responsive to a motion or movement of the subject. For instance, the motion data may capture or indicate information about acceleration, orientation, and other motion-related parameters that are responsive to a motion or movement of the subject, and are thereby usable to infer or derive mobility metrics of the subject. By processing this physical activity information, the machine-learning model is thereby able to generate a comprehensive set of mobility metrics.

In some examples, the plurality of mobility metrics comprises at least one of: an activity classification, an activity level, a walking detection, a walking speed, a step rate , a posture, a posture transition, a fall detection probability, a gait parameter and/or a step detection.

In some examples, the sensor data comprises acceleration data. Acceleration data provides a measure of acceleration in at least one direction or axis. The present disclosure recognizes a direct correlation or relationship between acceleration data and mobility metrics. In particular, acceleration data provides information that directly indicates a quality or quantity of a mobility property of a subject.

In some examples, the sensor data comprises three-axis acceleration data, wherein three-axis acceleration data indicates a measure of acceleration in each of three orthogonal axes.

In some examples, the sensor data comprises an acceleration magnitude indicating a total magnitude of acceleration of at least one of the one or more sensors.

Incorporating acceleration magnitude provides a simplified measure of overall movement intensity. Experimental analysis has identified that the incorporation of an acceleration magnitude in sensor data processed by the machine-learning model improves the accuracy and robustness of predicting the plurality of mobility metrics.

In some examples, the sensor data comprises, for each of one or more sensor parameters, only a time-series of sensor values for the sensor parameter. This approach captures the temporal evolution of movement patterns, enabling in-built analysis of trends and changes over time. The movement or motion of a subject over time has an inherent link or relationship with a mobility of the subject, and thereby the mobility metrics. Use of a time-series of sensor values thereby improves the accuracy and reliability of predicting the plurality of mobility metrics.

In some examples, for each of the one or more sensor parameters, the time-series of sensor values is: a time-series of sensor values captured over a predefined period of time; and/or time-series of a predetermined number of sensor values. Standardizing the time-series data format improves consistency in analysis and facilitates comparison across different time periods or subjects.

In some examples, the machine-learning model comprises a plurality of model coefficients, wherein at least two of the plurality of mobility metrics are responsive to each model coefficient in a same subset of one or more of the plurality of model coefficients.

A model coefficient, sometimes labelled a weight or bias, represents a parameter within the machine-learning model that influences how input data is processed to generate output predictions. In the context of multi-task learning, at least a subset (e.g., all) of the model coefficients are shared across multiple tasks, which advantageously allows the machine-learning model to exploit or make use of common features or patterns relevant to predicting various mobility metrics. This sharing of coefficients across tasks helps to capture interdependencies between different mobility metrics and improve overall prediction efficiency and accuracy.

In some examples, the machine-learning model comprises a sequence of layers, including an input layer, an output layer and one or more intermediate layers between the input layer and the output layer; each intermediate layer is configured to receive input data from a preceding layer in the sequence of layers and provide output data to a following layer in the sequence of layers; at least one intermediate layer is configured to receive: as input data from a preceding layer, data derived from and responsive to all sensor data processed by the machine-learning model; and process the input data using a same filter or kernel to produce the output data for provision to a following layer.

This layered architecture of the machine-learning model allows for progressive feature extraction and transformation of the sensor data. The use of shared filters or kernels in intermediate layers promotes learning of common features across different mobility tasks, enhancing the model's generalization capabilities and facilitates the performance of an eavesdropping function for improving the accuracy of the mobility metrics.

In accordance with a proposed approach, there is provided a computer-implemented method for training a machine-learning model for processing sensor data to produce a plurality of mobility metrics for a subject. The method includes obtaining training sensor data comprising a plurality of training data segments comprising, for each of a plurality of training subjects: one or more sensor data segments captured by one or more sensors supported by an upper body of the respective training subject; and for each sensor data segment, a plurality of training mobility metrics each indicating a respective ground truth mobility metric for the respective training subject during or at a time at which the sensor data segment was captured; and training, using the training sensor data, the machine-learning model to produce a trained machine-learning model configured to process sensor data to generate a plurality of mobility metrics.

In some examples, training, using the training sensor data, the machine-learning model comprises iteratively: processing each sensor data segment to produce a plurality of predicted mobility metrics, each indicating a respective predicted mobility metric for the respective training subject during or at a time at which the sensor data segment was captured, wherein each predicted mobility metric corresponds to a respective training mobility metric; determining a measure of error between the plurality of predicted mobility metrics and the training mobility metrics; and modifying the machine-learning model responsive to the measure of error.

In some examples, each sensor data segment comprises three-axis acceleration data, wherein three-axis acceleration data indicates a measure of acceleration in each of three orthogonal axes to thereby define a vector of acceleration with respect to the three orthogonal axes.

In some examples, the method further comprises supplementing the training sensor data by, for at least one training data segment: for at least one sensor data segment of the training data segment: rotating the vector of acceleration around one or more of the three orthogonal axes to produce a rotated sensor data segment; setting, for the rotated sensor data segment, the respective plurality of training mobility metrics of the sensor data segment as the respective plurality of training mobility metrics for the rotated sensor data segment; and supplementing the training data segment with the rotated sensor data segment and the respective plurality of training mobility metrics for the rotated sensor data segment.

This data augmentation technique enhances the robustness of the model by introducing rotational variations. By artificially expanding the training dataset, the approach improves the model's ability to handle different sensor orientations and body positions whilst accurately predicting the plurality of mobility metrics.

In some examples, for each sensor data segment, the number of the plurality of training mobility metrics is less than the number of the plurality of mobility metrics generated by the trained machine-learning model.

This embodiment recognizes that there is an inherent relationship and link between different mobility metrics, such that improving the prediction of fewer of the mobility metrics will, nonetheless, improve the prediction of all of the plurality of mobility metrics. In this way, data that may have previously been considered unusable or unsuitable for training a single task machine-learning model designed for predicting a particular mobility metric can be exploited to improve the prediction of the same mobility metric.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system in which embodiments may be employed;
Fig. 2 illustrates a proposed method
Fig. 3 illustrates a portion of a proposed machine-learning model;
Fig. 4 illustrates a proposed machine-learning model;
Fig. 5 illustrates another proposed machine-learning model;
Fig. 6 illustrates a method for training a machine-learning model;
Fig. 7 illustrates a procedure for augmenting training sensor data;
Fig. 8 illustrates error metrics of a proposed machine-learning model; and
Fig. 9 illustrates error metrics of proposed machine-learning models.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for predicting a plurality of mobility metrics for a subject. Sensor data, produced by sensors supported by an upper body of the subject, is processed using a multi-task learning model to predict the plurality of mobility metrics.

Embodiments are based on the realization that there is a direct relationship between such sensor data and mobility metrics, providing a mechanism for measuring or defining a quantifiable physical property of the subject.

It will be appreciated that a mobility metric may take the form of numeric, categorical, or binary data, e.g., depending on the specific property or parameter of mobility being measured or assessed.

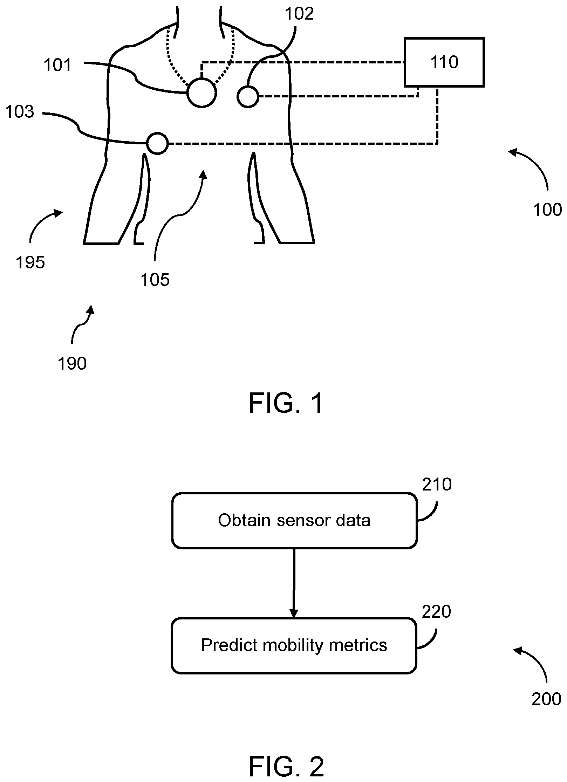

Fig. 1 illustrates a system 100 which embodiments may be employed, for improved contextual understanding. The system 100 comprises one or more sensors 101, 102, 103 (i.e. a sensor arrangement 105) and a processing system 110.

Each sensor 101, 102, 103 is positioned upon an upper body 195 of a subject 190. The one or more sensors are configured to generate sensor data, e.g. responsive to a motion, orientation and/or position of the upper body 195 of the subject 190.

For instance, any of the one or more sensors may be supported by a neck of the subject (e.g., on a pendant or lanyard); by an arm of the subject (e.g., strapped to an upper arm) and/or by a torso of the subject (e.g., adhered to the torso). Suitable examples of sensors will be provided later in disclosure.

The processing system 110 is configured to receive the sensor data from the one or more sensors 101, 102, 103. The processing system 110 processes the sensor data to produce a plurality of mobility metrics. Each mobility metric represents a physical property of the subject 190.

The present disclosure recognizes that more accurate and/or efficient identification of the mobility metrics can be achieved by processing the sensor data using a single machine-learning model (i.e. a shared machine-learning model) configured to perform a multi-task procedure. More specifically, the single machine-learning model produces the plurality mobility metrics for the subject.

The present disclosure proposes a concept of using a single machine-learning model trained to perform a multi-task function, e.g., using a multi-task learning (MTL) technique, to produce a plurality of mobility metrics. Each mobility metric therefore corresponds to a different task of the multi-task function (i.e., of a multi-task machine-learning model). An alternative label for a multi-task machine-learning model is a multi-task learning model or MTL model.

In this way, the present disclosure proposes an MTL model that is able to simultaneously predict multiple clinically relevant mobility metrics leveraging data from one or more sensors supported by an upper body of the subject.

Historically, tracking patient activity has required the use of multiple distinct algorithms, each dedicated to a specific metric, increasing deployment complexity and maintenance burden. The proposed MTL approach enables a single model to predict multiple activity metrics from wearable sensor data. This facilitates the use and exploitation of shared patterns across related tasks, thereby improving model generalization, increasing data efficiency and reducing overfitting. The use of a single MTL-based model (rather than separate individual algorithms) also helps simplifies maintenance and deployment, as only a single MTL-based model need be deployed and/or updated at a time, e.g., saving storage space and improving ease of upload.

For clinical integration, the sensor(s) used to produce sensor data would benefit from being unobtrusive, whilst reducing healthcare professional workload. The proposed approach, which exploits sensor data produced by one or more upper body worn sensors facilitates simultaneous prediction of multiple clinically relevant activity metrics (activity type, walking speed, and step rate) using sensor data from one or more unobtrusive and easily positioned sensors.

Fig. 2 illustrates a proposed computer implemented method 200, which may be performed by the processing system 110 (Fig. 1).

The method 200 comprises obtaining 210 sensor data produced by one or more sensors supported by the body of the subject.

Accordingly, the processing system may be communicatively coupled to each of the one or more sensors to receive the sensor data therefrom. Appropriate mechanisms for communicatively coupling two electronic components together are well known to the skilled person, may include any suitable wired or wireless communication channel (e.g. Bluetooth, Wi-Fi, Zigbee).

The method 200 further comprises processing 220 (at least) the sensor data using a single machine-learning model. As previously mentioned, the machine-learning model is trained using a multi-task learning technique, i.e., is a multi-task learning model (also known as an MTL model). Processing 220 the sensor data produces the plurality of mobility metrics for the subject.

In this way, the single machine-learning model (the MTL model) receives as input (at least) the sensor data induced by the one or more sensors and provides, as output, the plurality of mobility metrics for the subject.

In some examples, processing 220 the sensor data using a machine-learning model comprises processing the sensor data and one or more further instances of data for the subject (e.g., demographic data, medical history data and/or environmental data) using the single-machine-learning model to produce the plurality of mobility metrics for the subject.

In this way, the single machine-learning model (the MTL model) receives as input (at least) the sensor data induced by the one or more sensors and the one or more further instances of data for the subject. The single machine-learning model provides, as output, the plurality of mobility metrics for the subject.

Demographic data may comprise the subject's age, gender, height, weight, body mass index (BMI), ethnicity, or occupation. Medical history data may comprise information about pre-existing conditions, past surgeries, medications, or chronic illnesses. Environmental data may comprise information about factors such as temperature, humidity, terrain type, or indoor/outdoor location.

The method 200 may be iteratively repeated to process a larger series of input data produced by the sensor(s). In some cases, each instance of sensor data processed in one iteration of the method 200 may represent a windowed portion of the larger series. This approach allows for continuous analysis of sensor data over extended periods.

The larger series of input data may be an incoming stream of sensor data from the sensor(s) or a stored series of input data. When processing an incoming stream, the method 200 may be applied in real-time or near real-time to successive windows of sensor data as they are received. For stored data, the method 200 may be applied to sequential windows extracted from the stored series.

By iteratively applying method 200 to windowed portions of a larger dataset, the proposed method is able to track changes in mobility metrics over time. This approach facilitates the identification of trends, patterns, or anomalies in the subject's mobility that might not be apparent from a single isolated measurement.

The sensor data may comprise motion data responsive to a motion or movement of the subject. Motion data is indicative or processable to derive mobility metrics of the subject. The inclusion of motion data thereby improves the machine-learning model's ability to distinguish between levels or values for the mobility metric(s).

The sensor data may comprise acceleration data providing a measure of acceleration in at least one direction or axis. This acceleration data captures dynamic motion of the subject's upper body, which has a direct relationship with their mobility and therefore the mobility metrics. The inclusion of acceleration data in the sensor input thereby improves the machine-learning model's ability to distinguish between different types of movements and activities.

In particular, the acceleration data may comprise three-axis acceleration data, which indicates a measure of acceleration in each of three orthogonal axes. This type of data provides a representation of the subject's movement in three-dimensional space. The three orthogonal axes typically correspond to the x, y, and z directions, allowing for the capture of forward/backward, side-to-side, and up/down motions respectively.

Accordingly, the one or more sensors may comprise one or more accelerometers capable of measuring acceleration along one or more (e.g., three orthogonal) axes. The one or more accelerometers may be integrated into a single sensor or distributed across multiple sensors positioned on the upper body of the subject.

The sensor data may comprise an acceleration magnitude indicating a total magnitude of acceleration of at least one of the one or more sensors. This acceleration magnitude provides a scalar value representing the overall intensity of movement, regardless of direction, and may be calculated (if three-axis acceleration data is available) as the square root of the sum of squares of the acceleration components in each of three orthogonal axes (x, y, and z). Thus, the acceleration magnitude may be a magnitude of acceleration in a 3D environment.

In some examples, the sensor data may comprise one or more planar acceleration magnitudes (e.g., three planar acceleration magnitudes), each representing a magnitude of acceleration in a respective orthogonal plane. Each planar acceleration magnitude is another example of an acceleration magnitude. These acceleration magnitudes may provide a simplified representation of movement in one or more of an xy, yz, and xz plane. This form of sensor data captures planar motion patterns that could be relevant for certain mobility metrics.

Where the sensor data comprises both the (e.g., three-axis) acceleration data and the acceleration magnitude, the accuracy of the machine-learning model provides improved accuracy through separate processing of acceleration and magnitude signals.

When available, the sensor data may comprise angular velocity around the x-axis, y-axis, and/or z-axis from gyroscope measurements. In some examples, magnetic field strength measurements along the x-axis, y-axis, and/or z-axis from magnetometer data may be included as well.

In some examples, the sensor data may comprise barometric data (e.g., from a sensor functioning as a barometer). Barometric data may improve the detection of activities such as stair ascent or descent, e.g., improve an identification an activity classification.

The sensor data may comprise a segment or windowed portion of a larger time series of raw sensor data. This approach allows for the analysis of specific time intervals within the overall data collection period. By processing these segments or windows, the system may track changes in the plurality of mobility metrics over time, providing a dynamic view of the subject's mobility patterns and behaviors.

Correspondingly, in some examples, the sensor data comprises, for each of one or more sensor parameters, only a time-series of sensor values for the sensor parameter. By focusing on the temporal sequence of sensor values, the method captures the dynamic nature of human movement and posture changes over time. This temporal information is particularly useful for accurately predicting various mobility metrics, as many aspects of mobility are inherently time dependent.

The sensor data may include time-series of sensor values for various sensor parameters, such as: acceleration along the x-axis, acceleration along the y-axis, and/or acceleration along the z-axis. Additionally, total acceleration magnitude may be included as a parameter.

In some examples, for each of the one or more sensor parameters, the time-series of sensor values is: a time-series of sensor values captured over a predefined period of time; and/or time-series of a predetermined number of sensor values.

The standardization of time-series data for each sensor parameter improves the consistency and reliability of mobility metric predictions. By utilizing either a predefined time window (e.g., a 5ms window of sensor data) or a predetermined number of sensor values (e.g., 80 sensor values for each sensor parameter), the method ensures that the machine-learning model receives uniform input across different subjects and measurement sessions.

In some examples, the sensor data may comprise data produced by one or more sensors configured to be supportable only by a torso and/or neck of the subject. This reduces the risk of sensor data containing potentially misleading information that could be misinterpreted as mobility metrics. For instance, arm movements, which may not always be indicative of overall body mobility, would introduce noise or inaccuracies (i.e., information irrelevant to a mobility metric) into the sensor data if sensors were placed on the arms. Focusing on torso or neck-mounted sensors thereby provides a more stable and representative dataset for assessing the subject's mobility metrics.

Experimental analysis has identified that processing sensor data produced by one or more sensors fixed to a torso (i.e., torso-fixed sensors) improves the accuracy of the mobility metric prediction, e.g., compared to sensor data produced by one or more sensors carried by a pendant supported by the neck. This is attributable to, at least for acceleration data, larger variation in sensor acceleration patterns (in pendant-carried sensors) for the same activities due to sensor swing. Accordingly, in some examples, the sensor data may comprise data produced by one or more sensors securable to a torso of the subject.

Suitable examples of mobility metrics that may be produced using the proposed approach include: an activity classification, an activity level, a walking detection, a walking speed, a step rate; a posture, a posture transition, a fall detection probability, a gait parameter and/or a step detection. Other suitable examples will be readily apparent to the skilled person.

Activity classification categorizes the subject's action during the period in which (or at the time at which) the (raw) sensor data is captured. Example classifications for an activity classification include: sitting, standing, walking, running, ascending stairs, descending stairs, or performing other activities.

An activity level is a measure of the intensity or extent of a subject's physical activity, e.g., over a specific period. It may identify or quantify the degree of movement or exertion. An activity level can be defined using either a categorical or numeric metric, depending on the desired representation of the physical activity.

Walking detection identifies whether or not the subject is walking during the period in which (or at the time at which) the (raw) sensor data is captured. This metric is crucial for assessing mobility, e.g., and is relevant in clinical settings for monitoring patient recovery or in elderly care for fall risk assessment.

Walking speed quantifies how fast the subject is moving during the period in which (or at the time at which) the (raw) sensor data is captured. This metric is an indicator of functional capacity and can be used to track improvements in mobility or detect potential health issues.

Step rate, also known as cadence, measures or predicts the number of steps taken per minute. This metric provides information about the intensity and efficiency of the subject's gait, which is valuable for both clinical assessments and fitness tracking.

Posture assessment evaluates the subject's body position, such as whether they are upright, leaning, or lying down. Posture assessment may be expressed using either categorical (e.g., discrete classes such as "upright," "leaning," or "lying down,") or numeric data, (e.g., representing an angle or deviation from a reference position). This metric is important for monitoring sedentary behavior, assessing balance, and identifying potential risks for musculoskeletal issues.

A posture transition may indicate a change in the subject's body position, such as moving from sitting to standing or from standing to lying down.

A posture transition may comprise a binary value, a categorical value, and/or a numeric value. The binary value may indicate whether or not a transition has taken place during a given time period. The categorical value may indicate the specific postures involved in the transition, such as "sitting to standing" or "standing to lying down". The numeric value may represent the timing of the transition, expressible as a timestamp or as a relative time within the analyzed data window (if present).

A fall detection probability may represent the likelihood of a fall occurring based on the sensor data. This metric may be particularly valuable for monitoring elderly subjects or those with mobility impairments. The fall detection probability may be quantified as a numerical value between 0 and 1, where 0 indicates no risk of falling and 1 indicates a high likelihood of an imminent fall.

Gait parameters may include various measurable aspects of a subject's walking pattern. These may comprise metrics such as stride length, swing time, stance time, or gait symmetry. Thus, a metric for a gait parameter may comprise a value representing any one or more of these metrics.

Step detection may involve identifying individual steps taken by the subject. Thus, a metric of step detection may comprise one or more timestamps indicating when steps were detected. This information can be used to calculate step frequency, analyze walking patterns, and contribute to the assessment of overall mobility.

Suitable examples of multi-task machine-learning models, e.g., that may be trained using a multi-task learning technique, to process input data to produce output data resolving a plurality of different tasks are known in the art.

Typically, a multi-task machine-learning model comprises a plurality of model coefficients. These model coefficients, also known as parameters, biases, or weights, are numerical values that the model uses to process input data and generate predictions for each task.

**In** the proposed approach, a multi-task machine learning model shares or uses the same model coefficients across multiple separate tasks, which allows the model to exploit commonalities between different mobility metrics to improve overall prediction accuracy and efficiency compared to separate single-task models.

Thus, in some examples, the machine-learning model comprises a plurality of model coefficients, wherein at least two of the plurality of mobility metrics are responsive to each model coefficient in a same subset of one or more of the plurality of model coefficients.

By allowing multiple tasks to influence or be resolved using the same model coefficients, the proposed approach is able to more accurately capture or represent underlying relationships between various aspects of mobility. For example, coefficients related to detecting rapid acceleration might be relevant for both walking speed estimation and activity classification tasks. This model coefficient sharing not only improves computational efficiency by reducing the total number of unique coefficients but also improves the model's ability to generalize across tasks. It allows the model to transfer knowledge gained from one mobility metric to improve predictions for another, leading to more robust and accurate performance.

In some examples, the machine-learning model comprises a sequence of layers, including an input layer, an output layer and one or more intermediate layers between the input layer and the output layer. Each intermediate layer is configured to receive input data from a preceding layer in the sequence of layers and provide output data to a following layer in the sequence of layers.

This form of architecture for a machine-learning model is commonly called a neural network architecture. Similar to how biological neurons receive signals from other neurons, process them, and transmit signals to subsequent neurons, each layer in the sequence of layers defines a set of artificial neurons that receives and weights inputs (e.g., using a model coefficient), applies a function or filter, and passes the result to (neurons in) the next layer.

The input layer receives the raw sensor data. The intermediate layers, also known as hidden layers, perform feature extraction and transformation. The output layer is responsible for producing the final predictions for each of the mobility metrics. For a multi-task learning setup, this layer defines multiple task-specific outputs, each corresponding to a different mobility metric. The use of a shared architecture (e.g., up to the output layer) allows the model to learn common features that are relevant across multiple mobility metrics, potentially improving overall performance and generalization.

In some examples, at least one of the sequence of layers is a fully connected layer, such as a dense layer. For instance, the input layer may comprise a dense layer to process the initial sensor data. Thus, each piece of output data (for a subsequent intermediate layer) produced by the input layer will be derived from all input sensor data. This allows the model to capture complex relationships and interactions between different sensor inputs right from the initial processing stage.

At least one of one or more intermediate layers may be configured to receive as input data from a preceding layer, data derived from and responsive to all sensor data processed by the machine-learning model; and process the input data using a same filter or kernel to produce the output data for provision to a following layer. This shared processing approach may enable the model to identify common features across different mobility metrics, improving overall performance and generalization. The value(s) of the filter or kernel define at least some of the model coefficients of the machine-learning model.

The one or more intermediate layers may comprise one or more convolutional layers. In a convolutional layer, a filter (also known as a kernel) may be defined as a small matrix of weights (an example of a subset of model coefficients) that slides across the input data (to the intermediate layer), processing different sections or segments of said input to produce output data. This sliding window approach allows the filter to detect specific patterns or features at different positions locations within the input data.

As the filter moves across the input data, it performs element-wise multiplication with the current input segment and sums the results. This operation, known as convolution, produces a single output value for each position of the filter. The filter's movement is typically controlled by a stride parameter, which determines how many steps the filter takes between each movement of the filter.

The training of a machine-learning model that comprises one or more convolutional layers may comprise modifying or adjusting the weights (i.e., model coefficients) of the filter or kernel. This process allows the model to learn and adapt to the input data, improving its ability to extract relevant features.

The one or more intermediate layers may comprise one or more pooling layers, e.g., one or more max pooling layers.

In a pooling layer, the input data (to the pooling layer) is effectively downsampled to reduce its spatial dimensions. This operation helps to decrease computational complexity and mitigate overfitting. Max pooling, a common type of pooling, selects the maximum value within a defined region of the input data. For example, in a 2x2 max pooling operation, the layer may divide the input into 2x2 sections and output the maximum value from each section. This process may effectively reduce the size of the data while preserving the most prominent features. Other types of pooling include average pooling, which calculates the average value within each defined region, and min pooling, which identifies the minimum value within each defined region.

The one or more intermediate layers may comprise one or more rectifier layers, such as one or more Exponential Linear Unit (ELU) layers. a layer in a neural network that applies a non-linear activation function to its input. A rectifier layer uses a rectified linear unit (ReLU) or a variant (e.g., ELU) thereof as its activation function. This introduces non-linearity, helping to mitigate or reduce a risk of any vanishing gradients issue by maintaining activations near zero. For instance, an ELU layer performs an ELU activation function on each piece of input data (to the layer) to produce the output data.

The one or more intermediate layers may comprise one or more batch normalization layers. This is a layer that normalizes the input to a layer for each mini-batch. This normalization functions to adjust and scale the activations, to enhance stability and training speed.

The one or more intermediate layers may comprise one or more Gaussian noise layers. Such layers function to add (pseudo-)random noise drawn from a Gaussian distribution to input data (e.g., during training). This layer may help improve the robustness and generalization of the model by simulating variability in the input data. By way of working example, the or each Gaussian noise layer may have a standard deviation of 0.01.

The one or more intermediate layers may comprise one or more dropout layers. A dropout layer may (pseudo)randomly set a fraction of the elements of input data to 0 at each update during training. This technique helps prevent overfitting by reducing interdependent learning between neurons.

The one or more intermediate layers may comprise one or more flatten layers, which transforms multi-dimensional input data into a one-dimensional array or vector. A flatten layer may preserve all the information from its input while changing its shape, allowing subsequent layers to operate on a simplified representation of the data.

The output layer may comprise a flatten layer. This functions to convert any multi-dimensional feature representations learned by the preceding layers into a one-dimensional vector. This flattening process helps connect any convolutional and pooling layers, which may operate on multi-dimensional data, to the final output neurons that produce the individual mobility metrics.

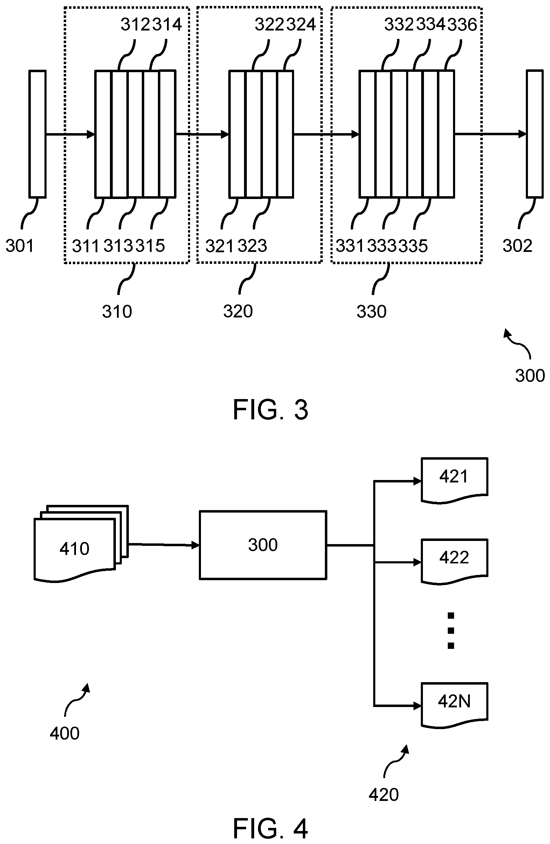

Fig. 3 provides an exemplary architecture for a portion 300 of a machine-learning model that may be employed to process (at least) the sensor data to produce the plurality of mobility metrics for the subject. This portion 300 may effectively represent a backbone block of the machine-learning model.

The portion 300 of the machine-learning model may comprise an input layer 301, an output layer 302 and plurality of intermediate layers comprising a sequence of convolutional blocks 310, 320, 330. Each convolutional block comprises a single convolutional layer 311, 321, 331 and further intermediate layers.

In the illustrated example, the sequence of convolutional blocks comprises a first convolutional block 310, a second convolutional block 320 and a third convolutional block 330.

In some examples, the convolutional layer 311, 321, 331 in each successive convolutional block comprises filters of increasing sizes. This approach allows the model to capture increasingly larger patterns or features in the data as it progresses through the network. For example, the first convolutional layer might use small filters to detect basic patterns like edges or simple movements, while later layers with larger filters can identify more complex motion patterns or postures.

In the illustrated example, the input layer 301 is a fully connected layer, in the form of a dense layer. A dense layer, also known as a fully connected layer, processes the input data in its entirety.

The first convolutional block 310 here comprises a sequence of layers formed from: a first convolutional layer 311, a max pooling layer 312, an ELU layer 313, a batch normalization layer 314 and a Gaussian noise layer 315.

The second convolutional block 320 here comprises a sequence of layers formed from: a second convolutional layer 321; a batch normalization layer 322; an ELU layer 323; and a Gaussian noise layer 324.

The third convolutional block 330 here comprises a sequence of layers formed from: a third convolutional layer 331; a batch normalization layer 332; an ELU layer 333; a max pooling layer 334; a Gaussian noise layer 335 and a dropout layer 336.

The output layer 302 is a flatten layer.

The proposed architecture for the portion 300 of the machine-learning model provides a balance between feature extraction (through the convolutional layers), data reduction (through the pooling layers) and data regularization (through the other layers). By combining these different layer types, the architecture defines an efficient framework for processing upper body sensor data to generate multiple mobility metrics simultaneously.

Fig. 4 provides an overview of an exemplary machine-learning model 400 that may be employed to process (at least) the sensor data to produce the plurality of mobility metrics for the subject.

The machine-learning model 400 comprises the portion 300 previously disclosed in Fig. 3. The machine-learning model comprises processing the sensor data 410 using the portion 300 to directly produce the plurality 420 of mobility metrics 421, 422, ..., 42N.

The portion 300 may be replaced by any other suitable architecture or configuration of layers that is able to process sensor data to produce the plurality of mobility metrics. For example, the portion 300 may be modified to include additional convolutional blocks, different types of layers, or alternative arrangements of layers.

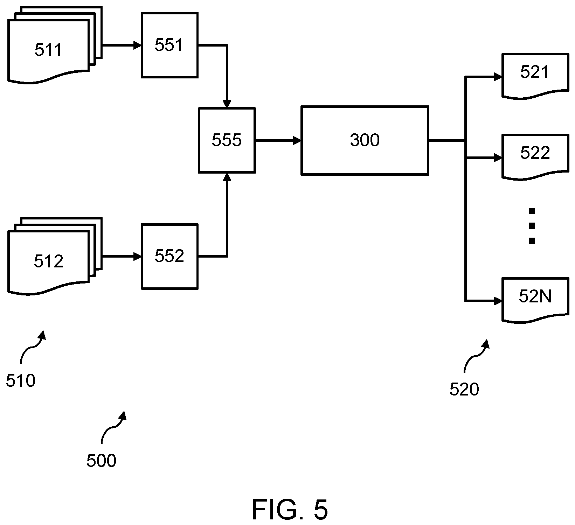

Fig. 5 provides an overview of another exemplary machine-learning model 500 that may be employed to process the sensor data 510 to produce the plurality 520 of mobility metrics 521, 522, ..., 52N for the subject.

The machine-learning model 500 comprises the portion 300 previously disclosed in Fig. 3.

The machine-learning model comprises pre-processing different parts 511 of the sensor data 510. For instance, a first part 511 may be processed using one or more first part convolution layers 551 and a second part may be processed using one or more second part convolution layers 552. This pre-processing approach may allow the model to extract specific features from different components of the sensor data before combining them in the main portion of the network.

By way of example, the first part 511 may correspond to (e.g., three-axis) acceleration data, while the second part 512 may represent an acceleration magnitude and/or from a different sensor type. By applying separate initial processing to these distinct data types, the model may be able to more effectively capture relevant features unique to each data component.

The pre-processed parts may be combined by a combination layer 555 before being input into the main portion 300 of the machine-learning model. For instance, the outputs from the first part convolution layers 551 and the second part convolution layers 552 may be concatenated. This concatenation process allows the model to preserve and utilize the distinct features extracted from different components of the sensor data. In other examples, the combination layer may employ other operations such as element-wise addition or multiplication to merge the pre-processed parts.

The combined output from the combination layer 555 serves as input to the portion 300, enabling the subsequent layers to process features derived from all parts of sensor data. This approach improves the model's ability to capture complex relationships between different types of sensor data and improve overall performance in generating mobility metrics.

Embodiments make use of a machine-learning model trained using a multi-task learning technique. For the purposes of a more complete understanding, a brief description of a suitable learning technique will be hereinafter provided.

As a general principle, training a machine-learning model typically first comprises obtaining a training dataset comprising a plurality of training data segments. Each training data segment comprises a training input data entry and a corresponding training output data entry.

In the context of the present disclosure, each training input data entry comprises a respective sample or segment of sensor data, i.e., a sensor data segment. Each training output data entries corresponds to the ground truth mobility metrics for each corresponding sample of sensor data of the corresponding training input data entry. Thus, each training output data entry is a plurality of training mobility metrics each indicating a respective ground truth mobility metric for the respective training subject during or at a time at which the sensor data segment was captured.

It will be appreciated that each training input data entry may, in some examples, further comprise an instance of additional data or information about the subject associated with the sensor data (e.g., demographic data, medical history data, and/or environmental data). This supplementary information may enhance the model's ability to generate more accurate and context-aware mobility metrics.

Training may then be performed by performing a series of training epochs. Each training epoch comprises processing each training input data entry using a machine-learning model to generate predicted output data entries. A measure of error (also known as a loss, cost or simply an error) between the predicted output data entries and corresponding training output data entries is used to modify the machine-learning model. For example, where the machine-learning model is formed from a sequence of layers representing neurons, (weightings of) the mathematical operation of each neuron may be modified until the error converges. Known methods of modifying a neural network include gradient descent, backpropagation models and so on.

For the first training epoch, an initialized version of the machine-learning model is employed. For each subsequent training epoch, the modified machine-learning model (produced as a result of the previous training epoch) is used in said training epoch.

The training epochs may be repeated until the error converges, and the predicted output data entries are sufficiently similar (e.g. ±1%) to the training output data entries. This is commonly known as a supervised learning technique. In some instances, training may be terminated after a fixed number of training epochs.

To train a model using a multi-task training technique, one approach may be to calculate (in each training epoch) an error (i.e., a measure of error) using a respective individual task loss (i.e., produced using a respective task-specific cost function) for each task of the machine-learning model. In this context, each task is the generation of a respective mobility metric. As such, an individual task loss represents an error in predicting the value(s) of a respective mobility metric by the machine-learning model.

The multi-task learning approach may thereby use a respective task-specific cost function for each task of the machine-learning model to calculate a loss (the individual task loss) for each task. The loss of a task represents an accuracy of the (current iteration of the) machine-learning model is performing the task, e.g., an accuracy of the predicted mobility metric produced by the machine-learning model for the task. The multi-task learning approach will also employ a combined cost function that integrates or combines these individual task losses to produce the error for training the machine-learning model. This method allows the model to learn shared representations across tasks while also optimizing or configuring for task-specific performance.

Each task-specific cost function thereby quantifies, in the form of the individual task loss, the error or discrepancy between the model's predictions and the ground truth for each particular task (i.e., each mobility metric). The choice of the cost function used for each task-specific cross-function may depend on the nature of the task, e.g., upon the format of data taken by the corresponding mobility metric.

For instance, for classification tasks, a cross-entropy loss may be used, while for regression tasks, mean squared error might be more appropriate. Thus, where a mobility metric takes the form of numeric data, a mean-squared error cost function may be used. Conversely, where the mobility metric takes the form of categorical or binary data (i.e., the outcome of a classification task) a cross-entropy cost function may be more appropriate.

The combined cost function aggregates or combines the individual task losses functions to produce the error. This error thereby serves as the overall optimization objective for the multi-task machine-learning model.

One exemplary combined cost function may comprise performing a weighted sum of the individual task losses, as follows: ${L}_{com} = {\sum }_{i = 1}^{n} {w}_{i} . {L}_{i}$ (1)

Where L_{com} represents the combined loss (i.e., the error), Lᵢ represents the loss for the i-th task, wᵢ represents the weight assigned to the i-th task, and n is the total number of tasks.

The weights of the combined cost function allow for regularization and/or prioritization of the individual task losses during training. These weights may be fixed based on prior knowledge about task importance and/or known discrepancies in (average) magnitudes.

As a working example, consider a scenario where the plurality of mobility metrics includes: speed, step rate, activity classification, and a walking detection (e.g. defined using binary data). In such an example, the error L_{com} may be calculated using the following combined cost function: ${L}_{com} = {L}_{speed} + \frac{{L}_{SR}}{100} + {L}_{AC} + {L}_{WD}$ (2) where L_{speed} is a determined loss for speed (e.g. calculated using a mean squared error cost function), L_{SR} is a determined loss for the step rate (e.g. calculated using a mean squared error), L_{AC} is a determined loss for the activity classification (e.g. calculated using a categorical cross entropy function) and L_{WD} is a determined loss for the walking detection (e.g. calculated using a binary cross entropy function).

In the working example, the determined loss for the step rate is scaled by a factor of 100 to align with the magnitude of the other losses. This scaling is useful because step rate values are typically much larger than the other metrics (often in the range of 80-120 steps per minute), which may cause the step rate loss to dominate the combined loss function without normalization. By dividing the step rate loss by 100, the contribution of this task to the overall loss becomes proportionally balanced with the other tasks, ensuring that the model optimizes equally for all mobility metrics rather than prioritizing step rate prediction at the expense of other metrics.

This demonstrates how a combined cost function that employs a weighted sum is advantageous for producing an error that is able to more fairly reflect the importance of each task.

During the training process, the model parameters (e.g., its weights) may be updated to target reducing or minimizing this combined cost function using any suitable optimization techniques such as stochastic gradient descent or its variants.

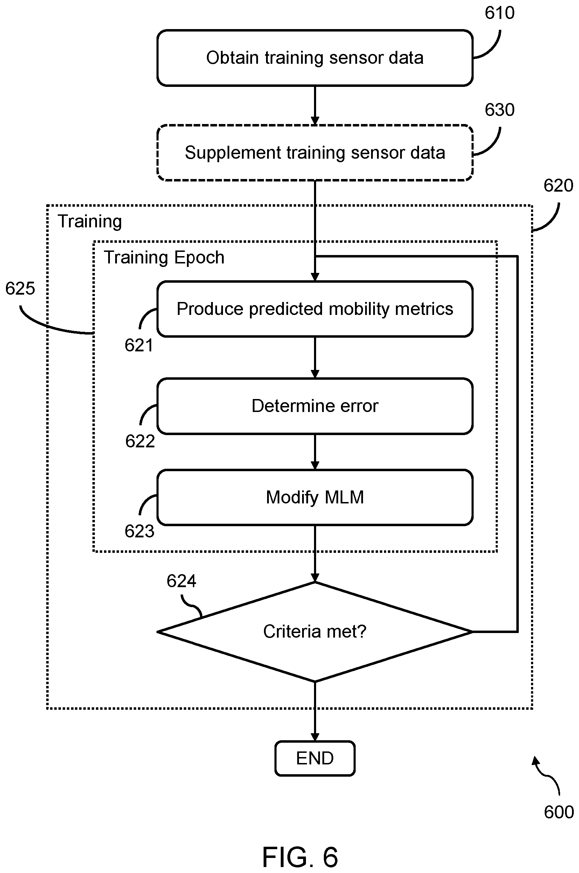

Fig. 6 illustrates a proposed computer-implemented method 600 for training a machine-learning model for processing sensor data to produce a plurality of mobility metrics for a subject. The method 600 may be performed by an appropriately configured processing system, as later described.

The computer-implemented method 600 comprises obtaining 610 training sensor data comprising a plurality of training data segments.

Each training data segment comprises, for each of a plurality of training subjects: one or more sensor data segments captured by one or more sensors supported by an upper body of the respective training subject; and for each sensor data segment, a plurality of training mobility metrics each indicating a respective ground truth mobility metric for the respective training subject during or at a time at which the sensor data segment was captured.

It will be appreciated that each training input data segment may, in some examples, further comprise additional information about the subject, such as demographic data, medical history, or environmental factors. Examples of such data have been previously provided.

The computer-implemented method 600 also comprises training 620, using the training sensor data, the machine-learning model to produce a trained machine-learning model configured to process sensor data to generate a plurality of mobility metrics.

Approaches for training the machine-learning model have been previously described, and may be readily employed to perform training 620 of the machine-learning model in method 600.

More particularly, training 620 the machine-learning model may comprise using any suitable multi-task learning technique herein described in the document. This approach allows the model to simultaneously learn to predict multiple mobility metrics, such as activity classification, activity level, walking detection, walking speed, step rate, posture, a posture transition, a fall detection probability, a gait parameter and/or a step detection from a single set of sensor inputs.

Fig. 6 provides an example technique for performing training 620, using the training sensor data, the machine-learning model. In particular, training the machine-learning model comprises iteratively performing a training epoch 625.

The training epoch 625 comprises processing 621 each sensor data segment to produce a plurality of predicted mobility metrics.

Each predicted mobility metric indicates a prediction of a respective mobility metric for the respective training subject during or at a time at which the sensor data segment was captured. Each predicted mobility metric corresponds to a respective training mobility metric.

The training epoch 625 also comprises determining 622 a measure of error between the plurality of predicted mobility metrics and the training mobility metrics. Approaches for determining a measure of error have been previously described, e.g., making use of individual task losses for each task.

The training epoch 625 also comprises modifying 623 the machine-learning model responsive to the measure of error. This process may comprise adjusting the model's coefficients, such as weights and biases, using optimization algorithms like stochastic gradient descent or its variants. The magnitude and direction of these adjustments may be determined by the calculated measure of error, with the goal of minimizing this error in subsequent iterations.

In some examples, training 620 the machine-learning model by iteratively performing the training epochs until one or more predetermined termination criteria are met. Exemplary predetermined termination criteria include: the error falling below a predetermined threshold error; the error failing to change by more than a predetermined percentage or value for more than a predetermined number of training epochs; and/or a predetermined number of training epochs being completed.

Accordingly, training 620 the machine-learning model may comprise determining 624 (e.g. after each training epoch or after a set number of training epochs) whether or not the one or more predetermined termination criteria have been met. Responsive to a positive determination (i.e. the one or more predetermined termination criteria are met), the training is terminated. Otherwise, a further training epoch (or set number of training epochs) is performed.

Suitable examples of the content for sensor data (i.e. and therefore each sensor data segment) have been previously disclosed.

In at least some examples, each sensor data segment comprises motion data, e.g., (e.g., three-axis) acceleration data. Motion data provides information about a motion or movement of the subject. Acceleration data provides a measure of acceleration in at least one direction or axis. Three-axis acceleration data indicates or provides a measure of acceleration in each of three orthogonal axes to thereby define a vector of acceleration with respect to the three orthogonal axes.

To improve the accuracy, robustness and versatility of the machine-learning model, it may be desirable to perform data augmentation of the training sensor data. In particular, augmenting the training sensor data using a modified version of at least a part of the training sensor data reduces the risk of overfitting.

It has been identified that the accuracy and robustness of the machine-learning model can be significantly improved, at least when the sensor data segments comprises acceleration data, by virtually rotating the acceleration data to produce modified versions of the sensor data segments.

Thus, the method 600 may comprise a process of supplementing 630 the training sensor data (before training 620).

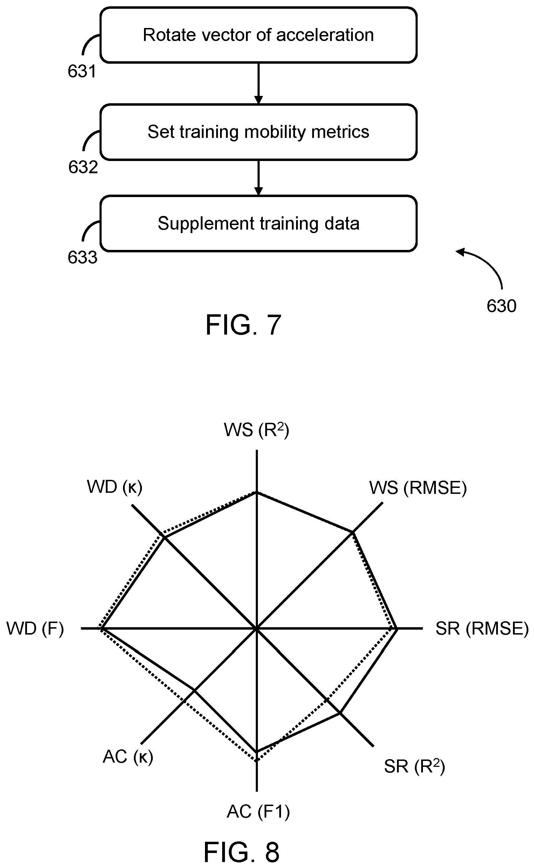

Fig. 7 illustrates one approach for supplementing 630 the training sensor data.

In particular, supplementing 630 the training sensor data may comprise, for at least one sensor data segment of each of at least one training data segment: rotating 631 the vector of acceleration around one or more of the three orthogonal axes to produce a rotated sensor data segment; setting 632, for the rotated sensor data segment, the respective plurality of training mobility metrics of the sensor data segment as the respective plurality of training mobility metrics for the rotated sensor data segment; and supplementing 633 the training data segment with the rotated sensor data segment and the respective plurality of training mobility metrics for the rotated sensor data segment.

The process thereby creates new sensor data segments by applying rotations to the original sensor data segments. The corresponding ground truth mobility metrics are preserved for these rotated segments. This approach operates under the assumption that the mobility metrics should remain consistent regardless of the sensor's orientation.

In one working example, for each sensor data segment processed in the supplementing 630 procedure, rotations of 6°, 10°, 15° and 20° around the X and Z axes of the acceleration data are formed, thereby producing an additional eight rotated sensor data segments for inclusion in the training sensor data for each sensor data segment. This is based on the recognition, during experimental analysis, that inclusion of rotated data segments produced by performing rotations around the Y-axis in the training sensor data resulted in negligible observable impact to the accuracy of the machine-learning model and the speed of convergence during training of the machine-learning model.

Turning back to Fig. 6, it is further herein recognized that the accuracy of the machine-learning model can be improved, across all tasks, when (further) training the machine-learning model using incomplete training sensor data. In particular, even if one or more of the predicted mobility metrics are absent in each sensor data segment, the machine-learning model still benefits from the available information. This approach allows for more efficient use of potentially incomplete datasets, which may occur in real-world scenarios where certain mobility metrics are not always available or measurable for every sensor data segment.

Accordingly, in some examples of method 600, for each sensor data segment, the number of the plurality of training mobility metrics is less than the number of the plurality of mobility metrics generated by the trained machine-learning model.

For example, in a clinical setting, certain mobility metrics like walking speed, step detection or step rate might not be measurable when the subject is confined to bed. However, other metrics such as posture, posture transition, activity level or activity classification could still be recorded. By incorporating these partially complete data segments into the training process, the model is able to learn to make predictions across a broader spectrum of scenarios, including those where only a subset of mobility metrics are available.

Fig. 8 illustrates the result of an experimental analysis of the accuracy of an MTL model compared to the accuracy of a set of STL models for predicting a plurality of mobility metrics.

The term "MTL model" refer to a machine-learning model that has been trained using a multi-task learning technique to produce a plurality of mobility metrics for the subject. Each mobility metric thereby corresponds to a different task of the multi-task learning technique. In particular, the MTL model has been trained using a herein proposed technique.

The term "STL model" refers to a machine-learning model that has been trained using a single-task learning technique to produce a single mobility metric for the subject. Thus, the number of STL models in the set of MTL models is equal to the number of tasks performed by the MTL model.

For the purposes of the experimental analysis, the plurality of mobility metrics comprises a walking speed (WS); a step rate (SR); an activity classification (AC); and a walking detection (WD).

Error metrics are generated for each mobility metric for both the MTL model and the set of STL models. The error metrics employed in this experimental analysis include an R² score; a (normalized) root mean squared error (RMSE); a κ score (also known as a Cohen's Kappa); an F-score; and or F1-score.

To produce the error metrics scores, each MTL model and STL model processes each sensor data segment in a test set of data segments. The MTL model generates predicted mobility metrics for each sensor data segment. The STL models each produce a respective mobility metric for each sensor data segment. The generated mobility metrics are then compared to known ground truth values for the sensor data segments of the test set. Error metrics are then computed by comparing the model's predictions to the ground truth labels across the entire test set, using well-established procedures for producing the error metrics.

Fig. 8 illustrates, after processing a test set of sensor data, the respective R² score for the walking speed (WS(R²)) and step rate (SR(R²)); the respective (normalized) root mean squared error (RMSE) for the walking speed (WS(RMSE)) and the step rate (SR(RMSE)); the respective κ score for the activity classification (AC(κ)) and the walking detection (WD(κ)); and the respective F-score or F1-score for the activity classification (AC(F1)) and the walking detection (WD(F)).

The dashed line represents the value of each error metric for the MTL model. The solid line represents the value of each error metric for the set of STL models. The closer to the center of the graph, the greater the accuracy of the corresponding prediction (i.e., the smaller the error).

Fig. 8 demonstrates that the MTL and STL models exhibit overall similar performance. Nonetheless, it is noted that the STL model demonstrates better performance for step rate estimation, while the MTL model performs better in activity classification and walking detection.

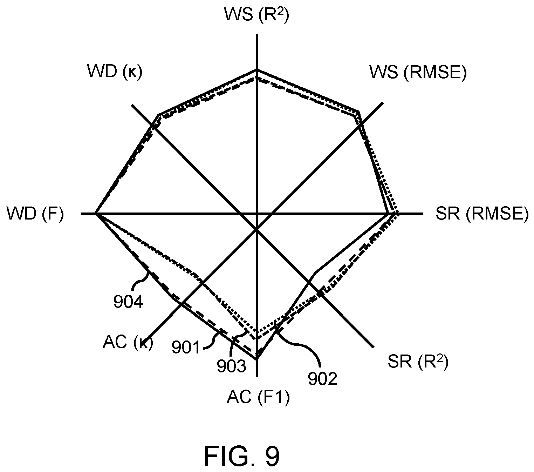

Fig. 9 illustrates the result of an experimental analysis of the accuracy of different MTL models for predicting a plurality of mobility metrics. Each MTL model is configured to process sensor data (in the form of three-axis acceleration data and an acceleration magnitude) to produce the plurality of mobility metrics.

The MTL models differ in the position of the sensor(s) that produce the three-axis acceleration data and the acceleration magnitude.

A first plot 901 (wide dashed line) represents an MTL model that processes sensor data produced by a sensor fixed on the chest of the subject(s). A second plot 902 (dotted line) represents an MTL model that processes sensor data produced by a sensor carried by a pendant on the side of the subject(s). A third plot 903 (short dashed line) represents an MTL model that processes sensor data produced by a sensor carried by a pendant on the front of the subject(s). A fourth plot 904 (solid line) represents an MTL model that processes sensor data produced by a sensor fixed on the left rib of the subject(s).

In a similar manner to Fig. 8, Fig. 9 illustrates, for each MTL model after processing a test set of sensor data, the respective R² score for the walking speed (WS(R²)) and step rate (SR(R²)); the respective (normalized) root mean squared error (RMSE) for the walking speed (WS(RMSE)) and the step rate (SR(RMSE)); the respective κ score for the activity classification (AC(κ)) and the walking detection (WD(κ)); and the respective F-score or F1-score for the activity classification (AC(F1)) and the walking detection (WD(F)).

Fig. 9 demonstrates that the MTL model generalizes well across all sensor locations, showing consistent performance overall. It is noted that sensor data produced by a left rib sensor presents slightly greater predictive accuracy for step rate, and slightly less walking speed predictive accuracy, compared to the other locations.

It is also noted that for the activity classification task, the two pendant sensors (front 903 and side 902) underperform compared to the body-fixed sensors (left rib 904 and chest 901).

Experimental analysis has also shown that, for at least tasks of walking detection and activity classification, a proposed multi-task machine-learning model trained according to a proposed approach achieves good levels of true-positive and true-negative detection.

**TABLE 1**

| | | | |
|---|---|---|---|
| True Label | Walking | 96.9% | 3.1% |
| | Not Walking | 1.3% | 98.7% |
| | | Not Walking | Walking |
| | | Predicted Label | |

Table 1 provides a confusion matrix for a walking detection task for an exemplary multi-task machine-learning model produced using a herein proposed approach. It can be identified that the model performs high accuracy prediction, albeit predicting walking activities slightly more accurately (98.7%) than non-walking activities (96.9%).

Table 2 provides a confusion matrix for an activity classification task for the same exemplary multi-task machine-learning model produced using a herein proposed approach. It can be identified that all activities are predicted with a high degree of accuracy (e.g., more than 90% of the time), except for the wheelchair class (64.2%).

These tables demonstrate the relatively high accuracy in performing a task achieved by the use of a proposed multi-task machine-learning model produced using a herein proposed approach.

**TABLE 2**

| | | | | | | | |
|---|---|---|---|---|---|---|---|
| True Label | Lying | 94.1% | 0.0% | - | 5.9% | 0.0% | 0.0% |
| | Stair Ascent | - | 85.5% | 3.7% | 0.2% | 8.6% | - |
| | Stair Descent | - | 1.5% | 91.3% | 0.1% | 7.1% | - |
| | Upright | 0.9% | 0.0% | 0.1% | 94.3% | 4.1% | 0.7% |
| | Walking | | 0.2% | 0.3% | 1.3% | 98.0% | 0.2% |
| | Wheelchair | 1.8% | - | - | 21.5% | 12.6% | 64.2% |
| | | Lying | Stair Ascent | Stair Descent | Upright | Walking | Wheelchair |
| | | Predicted Label | | | | | |

Turning back to Fig. 1, the skilled person would be readily capable of developing a processing system 110 for carrying out any herein described method. Thus, each step of a flow chart representing any method may represent a different action performed by a processing system, and may be performed by a respective module of the processing system.

Embodiments may therefore make use of a processing system 110. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

There is also provided a system 100 comprising the one or more sensors 101, 102, 103 and the processing system 110.

The one or more sensors 101, 102, 103 are configured to be supportable or supported by the upper body of the subject. For instance, the one or more sensors may be attached to or integrated into wearable devices such as pendants, chest straps, armbands, or adhesive patches. In some examples, the sensors may be incorporated into clothing items like shirts or vests. The sensors may be positioned on various parts of the upper body, including the chest, neck, shoulders, or upper back, to capture a range of motion and/or orientation data.

In some examples, the one or more sensors may be configured to be supportable only by a torso and/or neck of the subject. By limiting sensor placement to the torso or neck, the system reduces the risk of sensor data containing potentially misleading information that could be misinterpreted as mobility metrics. For instance, arm movements, which may not always be indicative of overall body mobility, would introduce noise or inaccuracies (i.e., information irrelevant to a mobility metric) into the sensor data if sensors were placed on the arms. Focusing on torso or neck-mounted sensors thereby provides a more stable and representative dataset for assessing the subject's mobility metrics.

This approach enhances the reliability of the collected data, leading to more accurate predictions of mobility metrics such as posture, activity classification, and walking detection. Additionally, torso or neck placement offers a more consistent frame of reference for acceleration and orientation measurements, which may be of use for tasks like estimating walking speed or step rate.

Experimental analysis has identified that processing sensor data produced by one or more sensors fixed to a torso (i.e., torso-fixed sensors) improves the accuracy of the mobility metric prediction, e.g., compared to sensor data produced by one or more sensors carried by a pendant supported by the neck. This is attributable to, at least for acceleration data, larger variation in sensor acceleration patterns (in pendant-carried sensors) for the same activities due to sensor swing.

Accordingly, in some examples, the one or more sensors may comprise one or more sensors securable to a torso of the subject.

It will be understood that disclosed methods are preferably computer-implemented methods. As such, there is also proposed the concept of a computer program (product) comprising code means for implementing any described method when said program (product) is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method for generating a plurality of mobility metrics for a subject, the computer-implemented method comprising:
obtaining sensor data produced by one or more sensors supported by an upper body of the subject; and
processing the sensor data using a single multi-task machine-learning model, to produce the plurality of mobility metrics for the subject, wherein each mobility metric corresponds to a different task of the multi-task learning technique.

2. The computer-implemented method of claim 1, wherein the plurality of mobility metrics comprises one at least one of: an activity classification, an activity level, a walking detection, a walking speed, a step rate, a posture, a posture transition, a fall detection probability, a gait parameter and/or a step detection.

3. The computer-implemented method of any one of claims 1 or 2, wherein the sensor data comprises three-axis acceleration data, wherein three-axis acceleration data indicates a measure of acceleration in each of three orthogonal axes.

4. The computer-implemented method of any one of claims 1 to 3, wherein the sensor data comprises an acceleration magnitude indicating a total magnitude of acceleration of at least one of the one or more sensors.

5. The computer-implemented method of any one of claims 1 to 4, wherein the sensor data comprises, for each of one or more sensor parameters, only a time-series of sensor values for the sensor parameter.

6. The computer-implemented method of claim 5, wherein, for each of the one or more sensor parameters, the time-series of sensor values is: a time-series of sensor values captured over a predefined period of time; and/or time-series of a predetermined number of sensor values.

7. The computer-implemented method of any one of claims 1 to 6, wherein the machine-learning model comprises a plurality of model coefficients, wherein at least two of the plurality of mobility metrics are responsive to each model coefficient in a same subset of one or more of the plurality of model coefficients.

8. A computer-implemented method for training a machine-learning model for processing sensor data to produce a plurality of mobility metrics for a subject, the computer-implemented method comprising:
obtaining training sensor data comprising a plurality of training data segments comprising, for each of a plurality of training subjects:
one or more sensor data segments captured by one or more sensors supported by an upper body of the respective training subject; and
for each sensor data segment, a plurality of training mobility metrics each indicating a respective ground truth mobility metric for the respective training subject during or at a time at which the sensor data segment was captured; and
training, using the training sensor data, the machine-learning model to produce a trained machine-learning model configured to process sensor data to generate a plurality of mobility metrics.

9. The computer-implemented method of claim 8, wherein training, using the training sensor data, the machine-learning model comprises iteratively:
processing each sensor data segment to produce a plurality of predicted mobility metrics, each indicating a respective predicted mobility metric for the respective training subject during or at a time at which the sensor data segment was captured, wherein each predicted mobility metric corresponds to a respective training mobility metric;
determining a measure of error between the plurality of predicted mobility metrics and the training mobility metrics; and
modifying the machine-learning model responsive to the measure of error.

10. The computer-implemented method of claim 8 or 9, wherein each sensor data segment comprises three-axis acceleration data, wherein three-axis acceleration data indicates a measure of acceleration in each of three orthogonal axes to thereby define a vector of acceleration with respect to the three orthogonal axes.

11. The computer-implemented method of claim 10, further comprising supplementing the training sensor data by, for at least one training data segment:
for at least one sensor data segment of the training data segment:
rotating the vector of acceleration around one or more of the three orthogonal axes to produce a rotated sensor data segment;
setting, for the rotated sensor data segment, the respective plurality of training mobility metrics of the sensor data segment as the respective plurality of training mobility metrics for the rotated sensor data segment; and
supplementing the training data segment with the rotated sensor data segment and the respective plurality of training mobility metrics for the rotated sensor data segment.

12. The computer-implemented method of any one of claims 8 to 11, wherein, for each sensor data segment, the number of the plurality of training mobility metrics is less than the number of the plurality of mobility metrics generated by the trained machine-learning model.

13. The computer-implemented method of any one of claims 1 to 12, wherein the machine-learning model comprises a plurality of model coefficients, wherein at least two of the plurality of mobility metrics are responsive to each model coefficient in a same subset of one or more of the plurality of model coefficients.

14. A computer program product comprising computer program code means which, when executed on a computing device having a processing system, cause the processing system to perform all of the steps of the method according to any one of claims 1 to 13.

15. A processing system configured to generate a plurality of mobility metrics for a subject, the processing system being configured to perform the method according to any one of claims 1 to 13.
